# EUROPEAN PATENT APPLICATION

(11) **EP 3 259 996 A1**
(43) Date of publication of application: **27.12.2017**
(21) Application number: 17176896.3
(22) Date of filing: 20.06.2017
(51) Int. Cl.: A23K 10/12, A23L 11/30, A23L 11/00, A23J 3/14

(54) **PROCESS FOR THE PRODUCTION OF FUNGAL BIOMASS, PRODUCT OBTAINABLE THEREBY AND USE OF FUNGAL BIOMASS PROTEIN**

(30) Priority: 21.06.2016 SE 1650877
(71) Applicant: CoMasa GmbH, 49477 Ibbenbüren (DE)
(72) Inventor: BERGMANN, Karl-Heinz, 53773 Hennef (DE); JÖNSSON, Bo, 49477 Ibbenbüren (DE)
(74) Representative: Awapatent AB

(57) **Abstract**

The present invention relates to a process for preparing a fungal biomass, such as fungal biomass protein, from legumes and recovering said fungal biomass protein from said legumes, where said process comprises certain steps including using a fungi chosen from *Aspergillus oryzae.*

A fungal biomass protein and a pure protein obtainable by said process as well as the use of a fungal biomass protein as vegtable protein in a food product are also described.

## Description

### Technical Field of the invention

The present invention relates to a process for preparing fungal biomass, especially fungal biomass protein, from legumes such as peas, beans, lentils, soybeans, clover, alfalfa, lupins, mesquite, carob and tamarind by using the fungi *Aspergillus oryzae.* The invention is also related to a fungal biomass protein and pure protein as obtained by the process of the invention and the use of such obtained fungal biomass protein and pure protein in food products and feed products, for human and animal consumption.

### Background Art

There is a need on the world market for more and more renewable proteins for use in food or feed products for human and animal consumption to replace existing protein sources, especially animal proteins.

There are different protein production processes available today, for instance during processing of certain vegetables, such as pea proteins, the proteins can be refined and extracted from the peas. During the pea protein process available today, one drawback is that pea starch is obtained in the process and is as well coming out as a large by-product. This overcapacity of pea starch is difficult to sell and re-use on the world market.

EP 2 040 567 discloses non-viable fish-feed comprising biomass from a species of Zygomycetes, wherein the biomass has been cultivated on lignocellulosic media such as spent sulphite liquid from the paper pulp preparation process.

US 7 186 807 discloses a process for extracting and refining the components of pea flour, i.e. the starch, the proteins, the internal fibres, and the solubles with the aid of equipment taken from a the potato starch factory. US2009/0259018 discloses a method for producing a protein concentrate from a starch containing grain. The fermentation organism used is a Rhizopus oryzae and Rhizopus microsporus.

There is a need within the technical field to increase, refine, extract and recover proteins, from any vegetable sources to replace the available animal proteins sources of today and to make available healthier alternatives. This would not only be beneficial to vegetarian people deciding actively to omit animal proteins but also for economic reasons and for health purposes.

### Summary of the invention

The present invention is related to a process for preparing a fungal biomass protein from legumes and recovering said fungal biomass protein and, in addition, recovering a pure protein from said legumes comprising the following steps:
a) providing a legume mixture comprising milled legumes and water;
b) heating the legume mixture to 70-80°C at pH 6-7 to obtain a heated legume mixture;
c) adding at least one enzyme chosen from amylases to the heated legume mixture and allowing the at least one enzyme to catalyse hydrolysis of carbohydrate present in the legume mixture;
d) increasing the temperature of the legume mixture to 100-125 °C at pH 5-6 to inactivate said at least one enzyme;
e) optionally adding water to the legume mixture;
f) adding a fungi chosen from the genera *Aspergillus oryzae* to the legume mixture in a bioreactor and allowing the fungi to react with the legume mixture for at least 6 hours at 30-40 °C to obtain a fungal biomass protein;
g) dewatering the legume mixture by removing water;
h) drying and milling the recovered fungal biomass protein to obtain a powder; and
i) recovering said fungal biomass protein and recovering said pure protein.

In another aspect, the invention relates to a fungal biomass protein and a pure protein obtainable by the process as obtained by above mentioned process.

In yet another aspect, the invention relates to the use of a fungal biomass protein as obtained in above mentioned process as vegtable protein in a food product or feed product for human and/or animal consumption.

### Detailed description of the invention

The present invention is related to a process for preparing a fungal biomass protein from legumes and recovering said fungal biomass protein and a pure protein from said legumes comprising the following steps;
In the step a) of the process, there is provided a legume mixture comprising milled legumes and water. The legumes are milled in an appropriate equipment for providing a size that is appropriate and optimized for large biomass yield. An appropriate size of the milled legumes is about 1 mm (fine powder). One option is that the legume is milled or provided in the form of a powder.
In step b) of the process, heating of the legume mixture to 70-80°C, eg at 72, 74, 75, 76, 78, at pH 6-7 to obtain a heated legume mixture is performed. A skilled person in the art may optimize the conditions in order to maximize outcome of the process.
In step c) of the process, adding at least one enzyme chosen from amylases to the heated legume mixture and allowing the at least one enzyme to catalyse hydrolysis of carbohydrate (starch) present in the legume mixture is performed. This makes the carbohydrate, i.e. starch, available for the fungi, to be added in the followng step. A skilled person in the art realizes that experimentation with the type of enzyme and amount of enzyme to optimize hydrolysis is possible. In step d), increasing the temperature of the legume mixture to 100-125 °C at pH 5-6 is performed to inactivate said at least one enzyme. A person skilled in the art may optimize the conditions and is routine skill in order to provide the optimum time and pH to inactivate the enzyme present.
In step e), optionally adding water to the legume mixture is performed. In step f), adding a strain of a fungi chosen from *Aspergillus oryzae* to the legume mixture in a bioreactor is performed and allowing the fungi to react with the legume mixture for at least 6 hours at 30-40 °C to obtain a fungal biomass protein. Any other suitable fungi which is able to react with the legume mixture is within the knowledge of a skilled person. The fungi is preferably recycled in the process for economic and environmental reasons. In step g), dewatering the legume mixture is performed by removing water. This may be performed in any conventional manner such as drying and/or dehydration by any means.
In step h), drying and milling the recovered fungal biomass protein to obtain a powder is performed and finally in step i) recovering said fungal biomass protein and additionally recovering said pure protein is performed. The fungal biomass protein and pure pea protein are obtained and separated from one another in connection with the drying step, see fig. 4.

By adding the at least one enzyme chosen from amylases to the heated legume mixture and allowing the at least one enzyme to catalyze hydrolysis of starch present in the legume mixture in step c), the starch in the legumes is made available to the fungi to be added in step f). The added fungi will cultivate and grow on the available starch, providing the biomass.

The legume starch volume that is coming out from the new process of the invention is so small due to the fact that the main part of the starch is used as cultivation feed for the fungi. Thus, the previous large amount of pea starch as obtained as a non-usable by product is now used for the fungi to cultivate on providing a protein rich fungal biomass. Thus, by the present invention, previous non-beneficial pea starch streams are made available for the fungi *Aspergillus oryzae* to cultivate on. Different types of fungi have been tested and it has been shown that Aspergillus oryzae is performing very well for biomass production.

Thus, the legume starch granules are opened up in step c) of the process and after that converted in the bioreactor to a protein rich fungal biomass. Thus, according to the invention it is a protein rich fungal biomass that is obtained. In addition, a pure protein existing in the legumes such as peas as one of the components is also recovered from the process.

Due to the process of the present invention, food and/or feed protein can be directly produced without any chemical additives and can more or less be directly used as human or animal food protein or further processed into real food products such as in bread, animal protein replacement and the like. In the complete production process only renewable raw material is used. In one embodiment of the invention an additional carbohydrate rich stream from another process could be added to the present process in order to work in cooperation with the present process.

In one embodiment of the invention, the process further comprises the step of recovering the heat in step d) and recycling the heat. This optional step saves energy and cost.

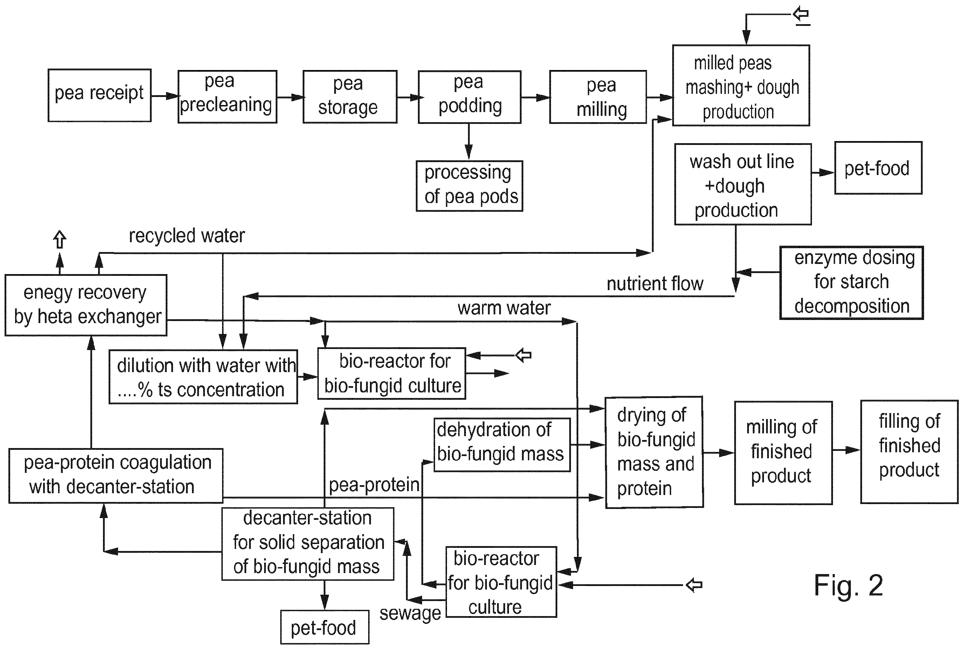

In an embodiment of the invention, the recovering of said pure protein, available as a component in the legumes, such as in peas, is performed in a separation step, see fig 2. Thus, two protein forms are obtained by the process of the invention, i.e. the fungi biomass protein and the pure legume protein. Both protein sources may be used together or separately as a vegetable protein alternative to animal protein and can be used from the range of animal feed to high end protein products.

In an embodiment of the invention, the at least one enzyme chosen from amylases, used in the process, is chosen from α-amylase, β-amylase and γ-amylase. The at least one enzyme may also be exchanged for any other enzyme being able to catalyze the carbohydrate as present in the legumes and making it available for the fungi to cultivate on.

In an embodiment of the invention, the recovered fungal biomass protein is further pelletized in an additional step j). Pellets may be beneficial from a production and transportation perspective. Pellets may further be beneficial for the further process.

In an embodiment of the invention, the fungal biomass protein as obtained in step f) is additionally washed in a washing step after the bioreactor.

In an embodiment of the invention, the process for producing biomass includes the step of aeration during biomass production.

In an embodiment of the invention, said at least one enzyme chosen from amylases is allowed to catalyze hydrolysis of the carbohydrate present in the legume mixture for at least 10 minutes.

In an embodiment of the invention, an additional carbohydrate rich stream, derived from another vegetable stream having a high carbohydrate content, is connected to the process in any of the steps a) - f). Thereby, more carbohydrate e.g. starch may be present for the fungi to cultivate on.

In an embodiment of the invention, the amount of dry solids of the legume mixture as started out with is in the range of 0.35 - 65 % by weight, preferably 1-60%, 5-50%, 10-40%, 15-30%, 20-25 % by weight.

In an embodiment of the invention, the process is a batch process, semi-batch process or continuous process.

In an embodiment of the invention, the legume as used in the legume mixture in the process is chosen from peas, beans, lentils, soybeans, clover, alfalfa, lupins, mesquite, carob and tamarind. The legumes may be processed before use, fresh or processed to a powder. It is naturally understood that any vegetable may be used in the process of the invention as long as it is compatible with the fungi *Aspergillus oryzae.*

In an embodiment of the invention, a fungal biomass protein and a pure protein is obtainable by the process as described herein. It is understood that the obtained protein may be used as a protein alternative in any kinds of products such as in food and feed for human and/or animal consumption.

Use of a fungal biomass protein as obtained in any one of claims 1-11 as vegtable protein in a food product.

Exemplary and available strains are *Aspergillus oryzae* var. oryzae CBS 819.72, *Fusarium venenatum* ATCC 20334, *Monascus purpureus* CBS 109.07, *Neurospora intermedia* CBS 131.92. Naturally any commercially available strain of the *Aspergillus oryzae* may be used herein. Thus, a few of the before said commercially available strains are available at CBS-KNAW Collections, at Uppsalalaan 8, 3584 CT, Utrecht, The Netherlands and at the American Type Collection (ATCC), respectively.

Thus, in view of the present process it will be possible to produce fungi biomass, such as biomass protein, in the amount of 10-20 000 tons per unit in an industrial scale. Thus, it is realized that the amount of produced biomass protein will have a large impact on protein replacement of today's animal protein. In addition, a novel use of the previously available by-product carbohydrate such as starch from legumes processes in industry will be available for biomass protein production through fungi.

### Brief description of the drawing

Fig. 1 discloses total dry biomass production harvested and dried after cultivation with the different disclosed fungi with (right hand bar) and without (left hand bar) the addition of enzyme. *Aspergillus oryzae* is the most effective among the tested fungi strains together with enzyme.
Fig. 2 discloses a process of the invention in the form of a flow sheet. The peas are first cleaned in a cleaning station. Before the production process is started, the peas are grained to the right particle size.
Fig. 3 discloses a table with data as obtained when testing the different fungal strains Aspegillus oryzae, Neurospora intermedia, Rhizopus Sp. Fusarium venenatum, Monascus purpureus. The biomass production produced is the highest for the fungi Aspergillus oryzae together with amylase enzyme and can be even further improved by aeration.

Due to a special enzymatic treatment the starch granules are opened up, and the starch is made available for the fungi. This means that the starch granules will be opened up and the carbohydrate is made available for the fungi. During this process step the starch will not be damaged and protein particles are made available, meaning that it is a soft and neutral process used having a very low impact on the end product. In a washing line the starch and pea protein are divided into two fractions. In another process step the pea protein is in a coagulation process recovered. The dry solids are picked out by a decanter. The equipment is designed for concentration from 0,35 % dry solids to 65 % dry solids and a pH value from 3.5 - 8,0 at the process temperature from 22 to 96 °C. The whole process can be controlled continuously but it can also be used in a batch process. The dewatering of the biomass is made over a sieve and a vacuum press together with decanter station. Thereby received dry solids are dried over a drying system, grained and then filled up in bags. In this new production process protein is obtained as the main product. An energy recovering system may be incorporated to reduce the energy consumption. By the use of the process, only slightly polluted waste water is in use, which mainly is recycled.

### Experiments

The objective of the experiment was to enrich the protein content of pea powder using edible *Ascomycetes* fungal strains.

The following *Ascomycetes* fungal strains were used: *Rhizopus* sp R15, *Aspergillus oryzae* var. oryzae CBS 819.72, *Fusarium venenatum* ATCC 20334, *Monascus purpureus* CBS 109.07, and *Neurospora intermedia* CBS 131.92

Firstly, the composition of pea powder was measured using standardized protocols or commercial kit, i.e. for starch which was analyzed using *Megazyme -* Total Starch Assay Kit and for protein: Standardized method of *Kjeldahl digestion method was* used to analyzed total nitrogen; and total crude protein calculated using a factor of 6.25
Structural carbohydrates and Ash: standardized method of NREL (National Renewable Energy Laboratory)

The results were as follows:

| | |
|---|---|
| Total Starch Assay Kit) | 50.97 ± 0.44 |
| Protein (analyzed using Kjeldahl method) | 16.45± 0.30 |
| Ash (analyzed using NREL method) | 2.69 ± 0.31 |
| Moisture | 9.53 ± 0.15 |

Different concentrations of pea powder were dissolved in distilled water and screened to determine its solubility post sterilization in order to screen for optimum substrate loading.

| Substrate loading | After autoclaving at 121°C (15 min) |
|---|---|
| 1% | No coagulation |
| 2% | No coagulation |
| 3% | Partial Coagulation |
| 4% | Coagulation (become solid) |
| 5% | Coagulation (become solid) |

In conclusion 2% substrate loading was chosen to be optimum

Effect of enzymes on pea powder hydrolysis was thereafter tested. Various enzymes (α-amylases) were added to 2% pea starch medium (sterilized) and incubated at 35°C for 24h. The amount of enzyme added was: 0.1 mL of Amylase 3000 units /mL activity

### Fermentation with fungi

2g pea starch was dissolved in 100ml distilled water (in a 250ml Erlenmeyer flask). Initial pH of the media was 6.2 ± 0.2 and was adjusted to pH: 5.5 ± 0.1 prior to autoclaving. Fungal spores were added to media (after cooling) to initiate the fermentation. Flasks were incubated at 35 °C in a water-bath for 36 h and samples collected at regular intervals (time 0, 6, 12, 24 and 36h). Enzyme addition: 0.1 mL α-Amylase 3000 units /mL activity (filter sterilized)

| Fungal strain | Spore count (spores / ml) |
|---|---|
| *Rhizopus* sp R15 | 3.8× 10⁶ |
| *Aspergillus oryzae* | 2.5× 10⁶ |
| *Fusarium venenatum* | 5.9× 10⁵ |
| *Monascus purpureus* | 3.9× 10⁵ |
| *Neurospora intermedia* | 2.9× 10⁶ |

### Measurement of total Biomass production

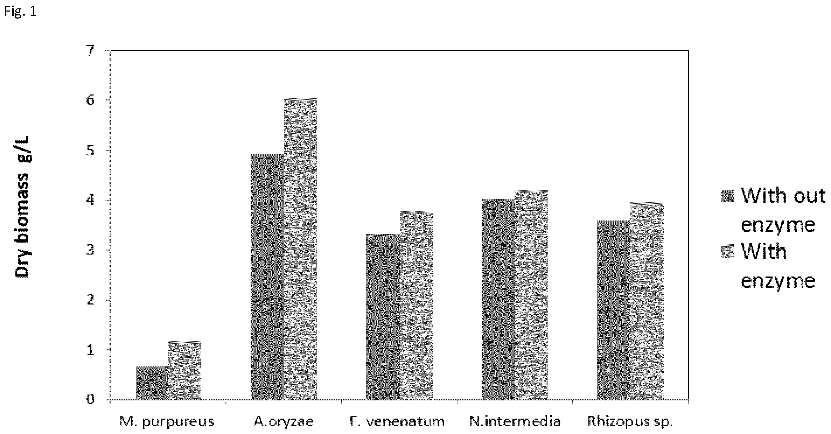

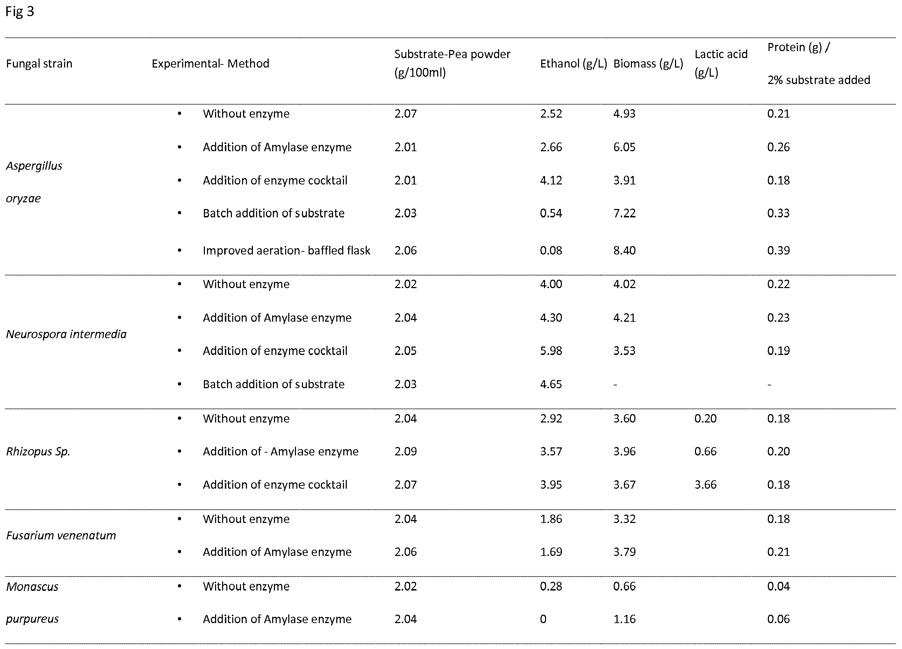

Biomass was harvested after 36h cultivation in 2% pea starch media with and without enzyme addition. Separation of biomass was achieved using a metallic mesh sieve. Biomass was washed with distilled water and dried at 70 ± 3°C and the dry weight recorded. See fig. 1 for results. The results show that *Aspergillus oryzae* was the best performing fungi strain for biomass production among the tested. The prior art fungi Rhizopus did not perform as well. The results of the experiments are shown in Fig 1 and Fig 3. In fig. 3 certain measurements of the tested fungi are shown.

### Conclusions

The studies revealed the potential of processed pea powder to be used as an efficient fungal cultivation media for specific fungi. The highest production of fungal biomass was shown by *Aspergillus oryzae.*

## Claims

1. A process for preparing fungal biomass protein from legumes and recovering said fungal biomass protein and, in addition, a pure protein from said legumes comprising the following steps:
a) providing a legume mixture comprising milled legumes and water;
b) heating the legume mixture to 70-80°C at pH 6-7 to obtain a heated legume mixture;
c) adding at least one enzyme chosen from amylases to the heated legume mixture and allowing the at least one enzyme to catalyze hydrolysis of carbohydrate present in the legume mixture;
d) increasing the temperature of the legume mixture to 100-125 °C at pH 5-6 to inactivate said at least one enzyme;
e) optionally adding water to the legume mixture;
f) adding a fungi chosen from *Aspergillus oryzae* to the legume mixture in a bioreactor and allowing the fungi to react with the legume mixture for at least 6 hours at 30-40 °C to obtain a fungal biomass protein;
g) dewatering the legume mixture by removing water;
h) drying and milling the recovered fungal biomass protein to obtain a powder; and
i) recovering said fungal biomass protein and, in addition, recovering said pure protein.

2. A process according to claim 1, wherein the process further comprises the step of recovering the heat in step d) and recycling the heat.

3. A process according to any one of claims 1-2, wherein the recovering of said pure protein is performed in a separation step after the dewatering step g).

4. A process according to any one of claims 1-3, wherein the at least one enzyme chosen from amylases is chosen from α-amylase, β-amylase and γ-amylase.

5. A process according to claim 1, wherein the recovered fungal biomass protein is further pelletized in a step j).

6. A process according to any one of claims 1-5, wherein the fungal biomass protein as obtained in step f) is additionally washed in a washing step after the bioreactor.

7. A process according to any one of claims 1-6, wherein said at least one enzyme chosen from amylases is allowed to catalyse hydrolysis of the carbohydrate present in the legume mixture for at least 10 minutes.

8. A process according to any one of claims 1-7, wherein an additional carbohydrate rich stream is connected to the process in any of the steps a) - f).

9. A process according to any one of claims 1-8, wherein the amount of dry solids of the legume mixture is in the range of 0.35 - 65 % by weight.

10. A process according to any one of claims 1-9, wherein the process is a batch process, semi-batch process or continuous process.

11. A process according to any one of claims 1-10, wherein the legume is chosen from peas, beans, lentils, soybeans, clover, alfalfa, lupins, mesquite, carob and tamarind.

12. A fungal biomass protein and a pure protein obtainable by the process as obtained by any one of claims 1-11.

13. Use of a fungal biomass protein as obtained in any one of claims 1 - 11 as vegtable protein in a food product.
